# EUROPÄISCHE PATENTSCHRIFT

(11) **EP 1 227 760 B1**
(45) Veröffentlichungstag und Bekanntmachung des Hinweises auf die Patenterteilung: **08.06.2005**
(21) Anmeldenummer: 00977439.9
(22) Anmeldetag: 30.10.2000
(51) Int. Cl.: A61B 17/12

(54) **VORRICHTUNG ZUR IMPLANTATION VON OCCLUSIONSWENDELN**
DEVICE FOR IMPLANTING OCCLUSION COILS
DISPOSITIF POUR L'IMPLANTATION D'ELEMENTS HELICOIDAUX D'OCCLUSION

(30) Priorität: 30.10.1999 DE 19952387; 09.03.2000 DE 10010840
(43) Veröffentlichungstag der Anmeldung: 07.08.2002
(60) Teilanmeldung: 04031018.7
(73) Patentinhaber: Dendron GmbH, 44799 Bochum (DE)
(72) Erfinder: MONSTADT, Hermann, 44797 Bochum (DE); HENKES, Hans, 45131 Essen (DE); DENK, Marion, 44803 Bochum (DE)
(74) Vertreter: Thiel, Christian, Dr. Dipl.-Chem.
(86) Internationale Anmeldenummer: PCT/EP2000/010660
(87) Internationale Veröffentlichungsnummer: WO 2001/032085

(56) Entgegenhaltungen:
- WO-A-91/13592
- WO-A-99/09894
- US-A- 5 624 449
- US-A- 5 733 329
- US-A- 5 941 888

## Beschreibung

Die Erfindung betrifft eine Vorrichtung zur Implantation von elektrolytisch ablösbaren Occlusionswendeln in Körperhohlräumen oder Blutgefäßen.

Der Einsatz endovaskulärer Techniken zur Occlusion von Körperhohlräumen oder Gefäßen wie Arterien, Venen, Eileitern oder vaskulären Fehlbildungen wie z. B. vaskulärer Aneurysmen ist bekannter Stand der Technik. Die Occlusionswendel wird dabei in der Regel mit Hilfe eines endovaskulären Führungsdrahtes durch einen Katheter in den zu occludierenden Hohlraum eingeführt und deponiert.

Die zur Deponierung notwendige Abtrennung der Occlusionswendel gestaltet sich dabei technisch besonders problematisch, denn die hierzu notwendige Vorrichtung muß einerseits möglichst klein gehalten sein, um durch den geringen Durchmesser des Katheters an die Zielorte geführt werden zu können, andererseits muß sie eine zuverlässige Abtrennung der Occlusionswendel bewirken, da es sonst bei Rückführung des Katheters zu einer ungewollten Entfernung der Occlusionswendel aus dem zu occludierenden Hohlraum und somit zu Verletzung und/oder Ruptur der Hohlraum- oder Gefäßwandung kommen kann.

Mechanische Verfahren zur Abtrennung der Occlusionswendel von der Einführhilfe sind zwar mit einem geringen Zeitaufwand verbunden, die technisch bedingte Starrheit der Verbindung von Occlusionswendel und Einführhilfe erschwert jedoch die Implantateinbringung. Zudem birgt die durch die Starrheit bedingte geringe Belastbarkeit der Verbindung ein nicht unerhebliches Risiko der vorzeitigen Trennung von Einführhilfe und Occlusionsimplantat. Weiterhin muß bei der mechanischen Trennung von Einführdraht und Occlusionswendel Energie übertragen werden (in der Regel durch Rotation des Einführdrahtes), durch die auch das Implantat aus der gewünschten Position verlagert werden kann.

Die elektrolytische Ablösung von Edelstahl-Drahtspitzen im Blut während der Transkatheter-Elektrokoagulation von Blutgefäßen oder Fehlbildungen derselben, wurde zum ersten mal 1979 durch Thompson et al und McAlister et al beschrieben (Radiology 133:335-340, November 1979, AJR 132:998-1000, Juni 1979).

Darauf aufbauend beschreibt die EP 0 484 468 eine Vorrichtung zur Implantation einer Occlusionswendel, basierend auf der elektrolytisch korrodierbaren Ausbildung der Drahtspitze des Führungsdrahtes an der Verbindung zwischen Führungsdraht und Occlusionswendel. Diese Vorrichtung nutzt zwar auf elegante Weise die zur Elektrothrombierung an die als Anode dienende Occlusionswendel angelegte elektrische Spannung für die gleichzeitige Abtrennung der Drahtspitze und der daran befindlichen Occlusionswendel, sie weist jedoch ebenso wie die vorgenannte mechanische Abtrennung den Nachteil auf, daß nur Implantate vordefinierter Länge abgetrennt werden können. Es muß also in der Regel vor der unmittelbaren Implantateinbringung anhand der Größe des zu occludierenden Hohlraumes die Länge, d. h. longitudinale Ausdehnung der einzusetzenden Occlusionswendel vordefiniert werden. Da die unregelmäßige Ausbildung der zu occludierenden Körperhohlräume jedoch die Einschätzung der zur Füllung notwendigen Länge der einzusetzenden Occlusionswendel erschwert, kann es dazu kommen, daß zu lange oder zu kurze Occlusionswendein in den zu occludierenden Hohlraum eingeführt werden, was eine unvollständige Occludierung einerseits bzw. der Beschädigung oder Ruptur der Wandung des zu occludierenden Hohlraumes oder angrenzender Gefäße andererseits bedingen kann.

Ein weiterer Nachteil der elektrolytischen Ablösung der Führungsdrahtspitze ist die Tatsache, daß zur Ausbildung des Führungsdrahtes nur Materialien eingesetzt werden können, welche hohe Stabilität aufweisen, damit die sichere Führung des einzuführenden Occlusionsdrahtes durch den Führungsdraht möglich ist. Die Werkstoffauswahl für die Ausbildung der elektrolytischen Ablösestelle ist daher sehr eingeschränkt.

Bei den im Stand der Technik vorkommenden Vorrichtungen zur elektrolytischen Ablösung von Occlusionswendeln sind Occlusionswendel und Führungsdraht nicht aus einem Stück gefertigt, sondern in der Regel mechanisch miteinander verbunden. Diese Ausbildung birgt den Nachteil, daß zur Gewährleistung ausreichender Stabilität im proximalen Bereich des Führungsdrahtes und zwecks Ermöglichung der elektrolytisch korrosiven Auflösung der Drahtspitze im distalen Bereich des Drahtes der Führungsdraht mittels aufwendiger Schleifverfahren zur Spitze hin verjüngt werden muß. Dieser korrodierbare Bereich der Drahtspitze des Führungsdrahtes an der Verbindungsstelle zwischen Führungsdraht und Occlusionswendel kann jedoch zur Gewährleistung ausreichender Stabilität der Verbindungsstelle einen gewissen Mindestdurchmesser von etwa 0,05 mm nicht unterschreiten, da er einer hohen Biegebeanspruchung unterliegt. Die die Verbindungsstelle zwischen Occlusionswendel und Führungsdraht darstellende korrodierbare Drahtspitze ist daher recht starr und benötigt zur elektrolytisch korrosiven Auflösung relativ lange Zeiten.

Die DE 44 45 715 C2 beschreibt die durch einen Laserstrahl, der mittels einer mitgeführten Lichtleitfaser auf die zu spaltende Stelle des Implantates fokussiert wird, herbeigeführte Abtrennung einer Occlusionswendel von der Einführhilfe. Diese Vorrichtung erlaubt es, anhand des Füllungszustandes des zu occludierenden Hohlraumes während des Eingriffes die zur Füllung des Hohlraumes optimale Länge der Occlusionswendel abzutrennen. So kann selbst bei Verwendung von hinsichtlich der Länge einheitlichen Occlusionswendeln die zur Füllung des Hohlraumes optimal geeignete Länge abgetrennt und deponiert werden. Die zur Anwendung dieser Vorrichtung notwendige Technologie ist jedoch derzeit noch sehr kostenintensiv.

US-A-5941888 beschreibt eine Vorrichtung zur Implantation von durch Elektrolyse ablösbaren Occlusionswendeln. Die Occlusionswendel ist dabei an mehreren in Abständen voneinander angeordneten Stellen elektrolytisch korrodierbar ausgebildet, ohne dabei durchgehend elektrisch leitend zu sein.

Da der Stand der Technik bisher keine hinsichtlich Kostenaufwand und Sicherheit befriedigende Möglichkeit zur endovaskulären Deponierung von Occlusionswendeln in der jeweils optimalen Länge bietet, liegt der Erfindung die Aufgabe zugrunde, eine Vorrichtung bereitzustellen, die es auf möglichst kosten günstigem, effektivem und sicherem Wege erlaubt, Occlusionswendeln in der jeweils richtigen Länge in Körperhöhlen oder Gefäßen zu deponieren.

Diese Aufgabe wird erfindungsgemäß durch eine Vorrichtung zur Implantation von durch Elektrolyse ablösbaren Occlusionswendeln in Körperhohlräumen oder Blutgefäßen mit einer Spannungsquelle, einer Kathode, einem Katheter und einer in dem Katheter in Längsrichtung verschiebbaren als Anode dienenden Occlusionswendel gelöst, bei der die Occlusionswendel an mehreren in Abständen voneinander angeordneten Stellen elektrolytisch korrodierbar ausgebildet ist, so daß im Kontakt mit einer Körperflüssigkeit eine oder mehrere variabel dimensionierte Längen der Occlusionswendel durch Elektrolyse abgelöst werden können und die dadurch gekennzeichnet ist, daß die Occlusionswendel von proximal bis distal durchgehend elektrisch leitend ausgebildet ist. Die Integration einer Vielzahl von elektrolytisch korrodierbaren Stellen in die Occlusionswendel bietet gegenüber herkömmlichen Vorrichtungen zur elektrolytischen Ablösung von Occlusionswendeln den Vorteil, daß während eines Implantatvorganges nicht nur eine sondern mehrere Längen derselben Wendel nacheinander abgelöst und in den zu occludierenden Hohlraum eingebracht werden können. Das spart nicht nur Kosten und Zeit, sondern dient außerdem der weiteren Minimierung des Operationsrisikos.

Die Erfindung beruht auf Experimenten der Erfinder, die ergaben, daß bei Anlegen eines Stromes an eine erfindungsgemäße Vorrichtung sich überraschenderweise spezifisch die dem distalen Ende des Katheters am nächsten liegende elektrolytisch korrodierbar ausgebildete Stelle der Occlusionswendel durch Elektrolyse löst. Diese Spezifität basiert wahrscheinlich darauf, daß einerseits die sich im Katheter befindenden elektrolytisch korrodierbaren Stellen der Occlusionswendel durch den Katheter vom ionischen Medium isoliert sind, also keiner Elektrolyse unterliegen können und andererseits die Stromdichte aufgrund des nach distal zunehmenden Widerstandes in der Occlusionswendel von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf.

Die erfindungsgemäße Vorrichtung zur Implantation von Occlusionswendeln kombiniert wie keine im Stand der Technik bekannte andere Vorrichtung die Vorteile der Occlusionseffektivität mit operativer Sicherheit und Kostengünstigkeit. Die während des Implantationsvorganges bestimmbare Implantatlänge verhindert, daß für den zu occludierenden Hohlraum eine zu kurze Occlusionswendel eingebracht wird, die zur Bildung eines für den zu occludierenden Raum ungenügend großen Thrombus führt. Weiterhin wird verhindert, daß eine hinsichtlich des zu occludierenden Hohlraumes zu lange Occlusionswendel eingeschoben wird, was die Gefahr von Verletzungen oder Rupturen des zu füllenden Hohlraumes oder angrenzender Gefäße minimiert. Es handelt sich zudem bei der elektrolytischen Abtrennung von Occlusionswendeln um eine erprobte Technik, deren Parameter weitgehend bestimmt sind. Letztlich weist die erfindungsgemäße Vorrichtung zur Implantation von Occlusionswendeln den Vorteil auf, daß für die Massenfertigung günstige Einheitslängen an Occlusionswendeln verwendet werden können. Dies stellt einen Kostenvorteil gegenüber den bei der herkömmlichen elektrolytischen oder mechanischen Abtrennung verwendeten Occlusionswendeln vorbestimmter Länge dar, weil dazu unterschiedlich lange Occlusionswendeln konfektioniert werden müssen, die dann beim Implantationsvorgang in ihrer Gesamtheit durch Abtrennung der Drahtspitze in den zu occludierenden Hohlraum eingebaut werden.

Da die elektrolytisch korrodierbaren Stellen der erfindungsgemäßen Vorrichtung einen Teil der Occlusionswendel selbst darstellen und zudem in Vielzahl vorliegen, unterliegen sie im Vergleich zu den herkömmlichen, starren elektrolytisch lösbar ausgebildeten Verbindungen zwischen Führungsdraht und Occlusionswendel einer wesentlichen geringeren Biegebeanspruchung während des implantationsprozesses. Diese geringe Biegebeanspruchung ermöglicht den Einsatz von elektrolytisch korrodierbaren Stellen mit wesentlich geringeren Durchmessem als im Stand der Technik, was zu einer verbesserten und schnelleren elektrolytischen Ablösbarkeit der Occlusionswendel führt. Solche geringen Durchmesser von weniger als 0,5 mm sind durch dafür geeignete, z. B. mechanische Verfahren zu erreichen.

Ein weitere Vorteill der Ausbildung der elektrolytisch korrodierbaren Stellen der erfindungsgemäßen Vorrichtung in der Occlusionswendel selbst gegenüber der im Stand der Technik vorkommenden Ablösung der Führungsdrahtspitze ist die wesentlich größere Auswahl an Materialien, welche zur Ausbildung der korrodierbaren Ablösestellen eingesetzt werden können. Im Gegensatz zur herkömmlichen Ablösung der Führungsdrahtspitze, müssen die in der Occlusionswendel angesiedelten elektrolytisch korrodierbaren Stellen der erfindungsgemäßen Vorrichtung keine besonders große Stabilität aufweisen, es können daher auch weniger stabile, flexiblere Werkstoffe eingesetzt werden, sofern sie korrodierbar und körperverträglich sind.

Zweckmäßigerweise kann sich proximal an die Occlusionswendel eine als Führungsdraht ausgebildete Einführhilfe anschließen. Eine solche Ausführungsform hat den Vorteil, daß der Führungsdraht aus gegenüber der Occlusionswendel kostengünstigeren Materialien ausgebildet sein kann, zumal er nicht in Kontakt mit dem Körpergewebe kommt. Weiterhin wird die Ausgestaltung des Führungsdrahtes vorzugsweise so bestimmt, daß sie eine gute Steuerung der Occlusionswendel durch den Katheter erlaubt, was zur besseren Deponierbarkeit beiträgt.

Bei einer solchen Ausgestaltung der erfindungsgemäßen Vorrichtung sind Einführhiife und Occlusionswendel vorzugsweise durch Löt- und/oder Klebeund/oder Schweißvorgänge und/oder mechanische Verbindungen miteinander verbunden. Es handelt sich um im Stand der Technik bekannte Verbindungsverfahren, die sich durch Einfachheit und die Stabilität der so herbeigeführten Verbindung auszeichnen.

In einer weiteren Ausführungsform können Führungsdraht und Occlusionswendel der erfindungsgemäßen Vorrichtung als Teile des selben Drahtes ausgebildet sein. Diese Ausführungsform zeichnet sich durch besondere Stabilität aus und kann sehr kostengünstig sein, da vorgenannte Anbindungsverfahren des Führungsdrahtes mit der Occlusionswendel wegfallen.

In einer besonders vorteilhaften Ausführungsform der erfindungsgemäßen Vorrichtung ist die Occlusionswendel oder ein Teil derselben als Mikrowendel ausgebildet. Diese Ausbildung weist den Vorteil auf, daß eine erhöhte Oberfläche für die Thrombosierung bereitgestellt wird. Zum selben Zweck können auch andere Ausgestaltungen der Occlusionswendel eingesetzt werden, die die Oberfläche derselben vergrößern, so sind beispielsweise Ausgestaltungen denkbar, deren distales Ende sich aufzweigt.

Um eine möglichst schonende und effektive Ausfüllung des zu occludierenden Hohlraumes zu gewährleisten, ist eine Ausbildung der erfindungsgemäßen Vorrichtung vorteilhaft, bei der die Occlusionswendel oder ein Teil derselben unter elastischer Vorspannung steht, so daß sie nach Entlassung aus dem Katheter Windungen ausbildet. Diese Ausbildung erlaubt eine dichte und schonende Ausfüllung des zu occludierenden Hohlraumes, ohne daß die Occlusionswendel durch die Wandung des zu occludierenden Hohlraumes zur Ausbildung solcher Windungen geformt werden muß, was das Risiko einer Wandungs-Ruptur vermindert. Es kommt dabei durch die elastische Vorspannung zur Ausbildung sekundärer Windungen.

Zweckmäßigerweise können die elektrolytisch nicht-korrodierbaren Abschnitte der Occlusionswendel eines oder mehrere der folgenden Materialien enthalten: Edelmetalle oder Edelmetall-Legierungen, korrosionsbeständige Keramikwerkstoffe, korrosionsbeständige Kunststoffe, vorzugsweise Platinmetall-Legierungen.

Ebenso bevorzugt ist eine Ausbildung der erfindungsgemäßen Vorrichtung, deren Occlusionswendel an den elektrolytisch korrodierbaren Stellen eines oder mehrere der folgenden Materialien enthält: Keramikwerkstoffe, Kunststoffe, Nichtedelmetalle oder Legierungen derselben, vorzugsweise rostfreies Stahl

Hierbei sind insbesondere die nicht rostenden Stähle der Typen AISI 301, 303 oder 316 bzw. Untergruppen dieser Typen geeignet.

Die für die Ausbildung des Occlusionswendel verwendeten Keramikwerkstoffe und Kunststoffe sind elektrisch leitfähig.

In einer vorteilhaften Ausführungsform werden für die Ausbildung der elektrolytisch nicht-korrodierbaren Abschnitte an den Übergängen zu den elektrolytisch korrodierbaren Stellen Materialkombinationen gewählt, die dazu geeignet sind, Lokalelemente auszubilden. Auf diese Weise wird - unabhängig von der Verringerung des Durchmessers der korrodierbaren Stellen - die elektrolytische Ablösbarkeit der Occlusionswendel verbessert.

Hierbei eignen sich am besten Materialkombinationen, in denen zur Ausbildung der elektrolytisch korrodierbaren Stellen nicht rostende Stähle, vorzugsweise der Typen AISI 301, 304, 316 oder Untergruppen derselben, Ti oder TiNi-Legierungen oder Co-Basislegierungen mit einem oder mehreren der folgenden Edelmetalle bzw. Edelmetall-Legierungen: Pt, Pt-Metalle, Pt-Legierungen, Au-Legierungen oder Sn-Legierungen.

Der Einsatz der vorgenannten Materialien zur Ausbildung der elektrolytisch nicht-korrodierbaren Abschnitte und der elektrolytisch korrodierbaren Stellen der Occlusionswendel gewährleisten die spezifische elektrolytische Korrosion der Occlusionswendel an den dafür vorbestimmten Stellen.

Besonders vorteilhaft ist die Ausbildung von elektrolytisch korrodierbaren Stellen, welche zu beiden Seiten Lokalelemente ausbilden. Diese Ausführungsform der elektrolytisch korrodierbaren Stellen ist wesentlich korrosionsfreudiger und korrodiert daher wesentlich schneller als elektrolytisch korrodierbare Stellen, welche nur zu einer Seite ein Lokalelement ausbilden. Bevorzugt sind deshalb Materialkombinationen mit möglichst großem Abstand in der elektrochemischen Spannungsreihe. Auch dies ist ein Vorteil der erfindungsgemäßen Vorrichtung gegenüber den im Stand der Technik vorkommenden Vorrichtungen die zur Ablösung des Embolisierungsdrahtes die Spitze des Führungsdrahtes korrodieren, da in diesem Fall nur zu einer Seite, nämlich zur Seite des Embolisierungsdrahtes (in der Regel ein Platindraht) ein Lokalelement ausgebildet wird.

Die Form der elektrolytisch korrodierbaren Stellen ist zweckmäßigerweise unter funktionellen Gesichtspunkten ausgestaltet. So ist es in Hinsicht auf die Biegebeanspruchung vorteilhaft, die Form der elektrolytisch korrodierbaren Stellen an die Form der Occlusionswendel anzupassen und diese beispielsweise in die Windungen einer als Mikrowendel ausgebildeten Occlusionswendel zu integrieren. Andererseits hat die im wesentlichen gerade Ausbildung der elektrolytisch korrodierbaren Stellen den Vorteil, daß sie technisch unaufwendig ist. Zum Zwecke einer guten Verschieblichkeit der Occlusionswendel im Katheter ist dabei die Ausrichtung der im wesentlichen geraden elektrolytisch korrodierbaren Stellen in Längsachse der Occlusionswendel vorteilhaft.

Die elektrolytisch korrodierbaren Stellen der Occlusionswendel können dabei beispielsweise durch Formteile gebildet werden, die zwischen die elektrolytisch nicht korrodierbaren Anteile der Occlusionswendel gefügt werden. Diese Ausführungsform hat den Vorteil, daß dabei eine besonders große Vielzahl verschiedener Werkstoffe für die Ausbildung der elektrolytisch korrodierbaren Stellen und elektrolytisch nicht-korrodierbaren Abschnitte miteinander kombiniert werden können. Diese Ausführungsform weist weiterhin den Vorteil auf, daß die elektrolytisch korrodierbaren Stellen und die elektrolytisch nicht korrodierbaren Abschnitte modular und daher technisch unaufwendig zur Bildung von Occlusionswendeln variabler Längen aneinandergefügt werden können. Dies ist besonders einfach, wenn die elektrolytisch korrodierbaren Stellen und somit die sie bildenden Formteile im wesentlichen gerade ausgebildet sind.

Zweckmäßigerweise können die die elektrolytisch korrodierbaren Stellen bildenden Formteile mit den nicht-korrodierbaren Abschnitten durch Löt- und/oder Klebe- und/oder Schweißvorgänge verbunden sein. Ebenso können die die elektrolytisch korrodierbaren Stellen bildenden Formteile mechanisch an die elektrolytisch nicht korrodierbaren Abschnitte angefügt werden, z. B. durch Einspannung oder Einklemmung, sofern die elektrolytisch nicht korrodierbaren Abschnitte Ausnehmungen zur Aufnahme der Formteile aufweisen. Dies ist z.B der Fall bei elektrolytisch nicht korrodierbaren Abschnitten, die als einen inneren Hohlraum umschreibende Mikrowendeln ausgebildet sind. Die Formteile können so formschlüssig in diesen Hohlraum eingefügt und fixiert werden. Dabei kann es weiterhin zweckmäßig sein, wenn die die Formteile aufnehmenden Außenbereiche der als Mikrowendel ausgebildeten elektrolytisch nicht korrodierbaren Abschnitte verstärkt sind.

Es ist dabei besonders vorteilhaft, wenn die die elektrolytisch korrodierbaren Stellen bildenden Formteile durch Ätzen oder andere Verfahren vorkorrodiert sind, so daß sich ihr Durchmesser zur Mitte hin verjüngt. Die äußeren bzw. geschwächten Anteile der Formteile mit größerem Durchmesser werden dann durch beispielsweise artfremdes Verschweißen, mechanische Einbringung oder Verklebung an die elektrolytisch nicht korrodierbaren Abschnitte gefügt. Die Verbindung zwischen elektrolytisch korrodierbaren Stellen und elektrolytisch nicht korrodierbaren Abschnitten ist daher sehr stabil, wohingegen der sich infolge der Vorkorrosion zur Mitte des Formteils verjüngende Durchmesser zur guten elektrolytischen Ablösung der Occlusionswendel führt. Dabei ist für die Materialkombination von Platin-Legierungen oder Platin-Metallen als Werkstoff für die Ausbildung der elektrolytisch nicht korrodierbaren Abschnitte mit nichtrostendem Stahl als Werkstoff für die die elektrolytisch korrodierbaren Stellen bildenden Formteile die Verbindung durch artfremdes Verschweißen besonders bevorzugt. Für den Fachmann selbstverständlich ist auch die Möglichkeit der Vorkorrodierung der elektrolytisch korrodierbaren Stellen, wenn diese nicht aus Formteilen gebildet werden.

Es ist dabei zweckmäßig, die Formteile mit einer teilweisen Beschichtung eines Werkstoffs zu versehen, der in der Spannungsreihe über dem Material steht, welches die Formteile ausbildet. Diese Ausführungsform ist besonders vorteilhaft hinsichtlich elektrolytischen Korrodierbarkeit der elektrolytisch korrodierbaren Stellen, welche dort angesiedelt sind, wo die Beschichtung am Formteil fehlt. Als besonders vorteilhaft haben sich hierbei Beschichtungen von Zn oder Sn bzw. Legierungen dieser Metalle auf Formteilen aus nicht rostendem Stahl herausgestellt.

Die mechanische Anbringung der Formteile ist dabei besonders vorteilhaft, wenn die elektrolytisch korrodierbaren Stellen im wesentlichen gerade ausgebildet und entlang der Längsachse der Occlusionswendel angeordnet sein sollen. Die Aneinanderfügung der die Occlusionswendel bildenden Module (nicht elektrolytisch korrodierbare Abschnitte und elektrolytisch korrodierbare Stellen) ist in diesem Fall mit einem besonders niedrigen technischen Aufwand verbunden.

Zur weiteren Sicherung und Stabilisierung der Aneinanderfügung der einzelnen, die Occlusionswendel bildenden Module, kann auch eine Kombination der genannten Verfahren eingesetzt werden.

Die Flexibilität der Occlusionswendel ist auch bei mechanischer Aneinanderfügung aufgrund der Materialauswahl und aufgrund des geringen Durchmessers der die elektrolytisch korrodierbaren Stellen bildenden Formteile gewährleistet.

In einer Ausführung der erfindungsgemäßen Vorrichtung sind die die elektrolytisch korrodierbaren Stellen bildenden Formteile als Mikrosystembauteile ausgebildet. Diese können beispielsweise als längliche Mikrosystembauteile ausgebildet sein, deren Durchmesser sich zur Mitte hin verjüngt. Das Einfügen der Mikrosystembauteile erfolgt durch die genannten gängigen Verfahren. Die Verwendung solcher sich zur Mitte hin verjüngender Mikrosystemenbauteile weist dabei den Vorteil auf, daß die Bereiche des größten Durchmessers an die elektrolytisch nicht korrodierbaren Abschnitte gefügt werden und somit einen stabilen Zusammenhalt gewährleisten. Der sich verjüngende Bereich mit geringerem Durchmesser ist dagegen dem umgebenden Medium ausgesetzt und kann leicht elektrolytisch korrodiert werden. Auf diese Art und Weise können besonders geringe Durchmesser der elektrolytisch korrodierbaren Stellen erzielt werden.

Die Verjüngung des Durchmessers der elektrolytisch korrodierbaren Stellen zur Mitte hin ist hinsichtlich guter Korrodierbarkeit auch für andere Ausbildungen der elektrolytisch korrodierbaren Stellen zweckmäßig.

In einer weiteren Ausführungsform der erfindungsgemäßen Vorrichtung enthält die Occlusionswendel oder ein Teil derselben einen durchgängigen Drahtkern aus elektrolytisch korrodierbarem Material, welcher von einer in Längsachse in Abständen unterbrochenen Umhüllung aus elektrolytisch korrosionsbeständigem Material umgeben ist. Der Führungsdraht und der Kern der Occlusionswendel sind dabei vorzugsweise Teile desselben Drahtes. Diese Ausführungsform ist besonders kostengünstig, weil einerseits der zur Verbindung von Occlusionswendel und Einführhilfe notwendige Schweiß-, Löt- oder Klebevorgang entfällt und andererseits der Drahtkern aus in der Regel gegenüber den elektrolytisch nicht-korrodierbaren Materialien kostengünstigeren elektrolytisch korrodierbarem Material besteht, wohingegen nur geringe Mengen an gegenüber dem Drahtkern elektrolytisch schlechter korrodierbarem Material zur Beschichtung eingesetzt werden müssen.

Der Durchmesser der Occlusionswendel der erfindungsgemäßen Vorrichtung wird dabei zweckmäßigerweise so gewählt, daß er einerseits hinreichende Stabilität gewährleistet und andererseits die elektrolytisch korrodierbaren Steilen in situ elektrolytisch gut korrodierbar sind. Vorteilhaft in dieser Hinsicht sind Ausführungsformen mit an den elektrolytisch korrodierbaren Stellen vorliegenden Durchmessern der Occlusionswendel zwischen 0,01 bis 0,05 mm, vorzugsweise 0,02 bis 0,04 mm und besonders bevorzugt 0,03 mm. Die elektrolytisch nicht korrodierbaren Abschnitte der Occlusionswendel können dagegen auch größere Durchmesser aufweisen.

In einer weiteren vorteilhaften Ausführungsform ist die Spitze des Führungsdrahtes beispielsweise durch eine schlecht korrodierbare Materialbeschichtung oder Überzug mit einem Schrumpfschlauf isoliert, so daß sie nicht elektrolytisch korrodierbar ist.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln und/oder Tumorembolisation durch Thrombosierung bestimmt.

Die Erfindung wird nachfolgend beispielhaft anhand der in den Zeichnungen veranschaulichten Ausführungsbeispiele näher erläutert. Es stellen dar:
- Figur 1: die Vertikalansicht einer in ein Beerenaneurysma positionierten Mikrowendel 3 mit dazugehöriger Vorrichtung in vielfacher Vergrößerung;
- Figur 1b und 1c: die Ausschnitts-Vergrößerung 15 aus Figur 1 in dem Bereich der elektrolytisch korrodierten Stelle 14 der Mikrowendel 3 in zwei unterschiedlichen Ausbildungen der Mikrowendel 3;
- Figur 2a bis c: in gegenüber Figur 1 stärkerer Vergrößerung drei Möglichkeiten der Anordnung elektrolytisch korrodierbarer Stellen 11 und elektrolytisch nicht korrodierbarer Abschnitte 10 in der erfindungsgemäßen Mikrowendel 3;
- Figur 3a bis c: in gegenüber Figur 1 stärkerer Vergrößerung drei Möglichkeiten der Anordnung von elektrolytisch korrodierbarer Stellen 11 und elektrolytisch nicht korrodierbarer Abschnitte 10 in der erfindungsgemäßen Mikrowendel 3 mit entlang der Längsachse der Mikrowendel 3 angeordneten elektrolytisch korrodierbaren Stellen 11;
- Figur 4: Längsschnittansicht der Anbringung des Führungsdrahtes (2) an die Occlusionswendel.

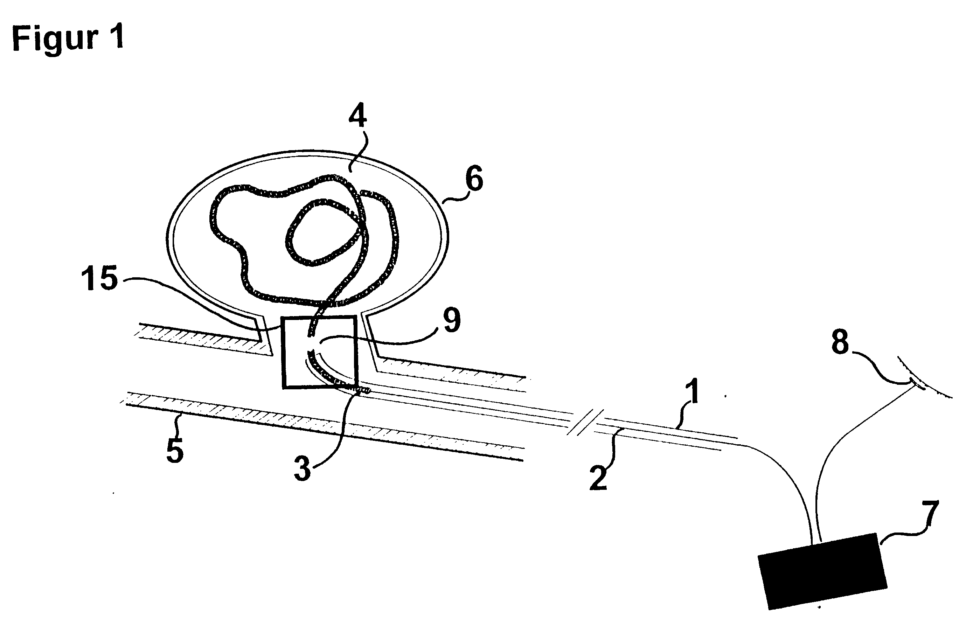

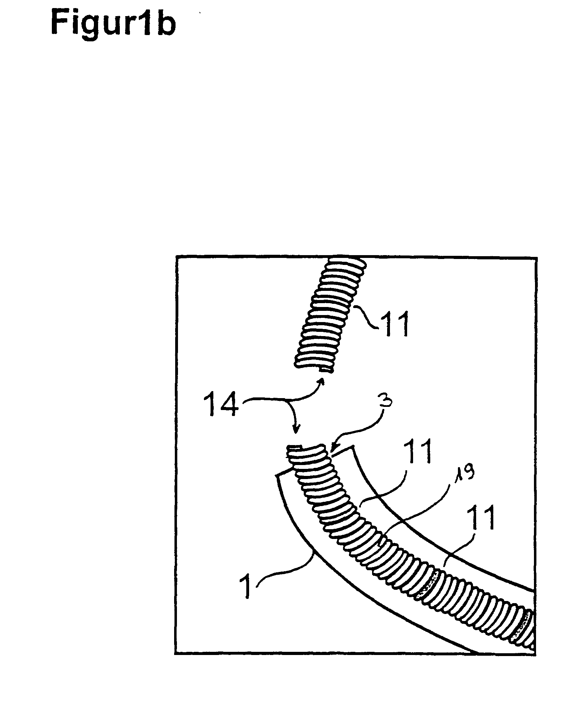

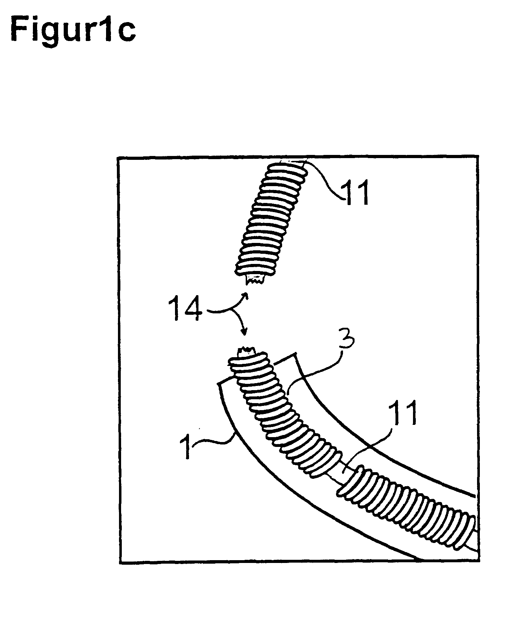

In der Figur 1 ist mit 1 ein Katheter, insbesondere ein biegsam ausgebildeter Mikrokatheter bezeichnet. Durch den Mikrokatheter 1 wird die aus einer Platinmetall-Legierung gefertigte, mit elektrolytisch korrodierbaren Stellen 11 aus Edelstahl versehene als Mikrowendel ausgebildete Occlusionswendel 3 mit Hilfe des schweißtechnisch an die Mikrowendel 3 gefügten Führungsdrahtes 2 an den Eingang des Aneurysmas 6 positioniert. Da diese durch artfremdes Schweißen gefügte Verbindung zwischen Führungsdraht 2 und Mikrowendel 3 nicht zur elektrolytischen Ablösung der Mikrowendel 3 bestimmt ist und folglich nicht von besonders geringem Durchmesser sein muß, ist sie besonders stabil ausgebildet. Die Verwendung von nichtrostendem Stahl und Platin für die Ausbildung des Führungsdrahtes einerseits bzw. der Occlusionswendel andererseits ist dabei besonders vorteilhaft, da der in Stahl enthaltene Nickel sich beim Verschweißen zu einer sehr glatten und stabilen Verbindung mit dem Platin fügt. Durch die in Längsachse des Mikrokatheters nach distal erfolgende Verschiebung der Führungshilfe 2 erfolgt die Einführung der Mikrowendel 3 in das Aneurysma 6, welche aufgrund ihrer elastischen Vorspannung nach Verlassen des Mikrokatheters 1 sekundäre Windungen 4 ausbildet. Durch die Längsverschieblichkeit von Führungsdraht 2 und Mikrowendel 3 im Mikrokatheter 1 wird eine an das Volumen des auszufüllenden Hohlraumes individuell angepaßte Länge der Mikrowendel 3 in diesen eingebracht. Anschließend wird mit Hilfe der Spannungsquelle 7, der auf der Körperoberfläche positionierten Kathode 8 und der als Anode dienenden im zu occludierenden Aneurysma 6 positionierten Mikrowendel 3 eine Spannung über einen Zeitraum von 0,1 - 20 Minuten angelegt. Dadurch wird die elektrolytische Abtrennung des sich im Blut befindenden Teils der Mikrowendel 3 an der dem distalen Katheterende nächstliegenden elektrolytisch korrodierbaren Stelle 9 aufgelöst. Figur 1 stellt eine Mikrowendel 3 dar, deren dem distalen Ende des Mikrokatheters 1 nächstliegende elektrolytisch korrodierbare Stelle 9 bereits elektrolytisch korrodiert wurde.

Die Figur 1b stellt in Ausschnittsvergrößerung der Figur 1 die dem distalen Ende des Mikrokatheters 1 nächstliegende elektrolytisch korrodierbare Stelle in korrodiertem Zustand 14 dar. Weitere, sich im Blut oder noch im Mikrokatheder 1 befindende elektrolytisch korrodierbare Stellen 11 liegen dagegen noch in intaktem Zustand vor. Die elektrolytisch korrodierbaren Stellen sind an die Form der Mikrowindungen 19 der Mikrowendel angepaßt.

Die Figur 1c stellt ebenfalls in Ausschnittsvergrößerung der Figur 1 die dem distalen Ende des Mikrokatheters 1 nächstliegende elektrolytisch korrodierbare Stelle in korrodiertem Zustand 14 für eine Mikrowendel 3 mit im wesentlichen geraden elektrolytisch korrodierbaren Stellen 11 dar, die an der Längsachse der Mikrowendel 3 ausgerichtet sind.

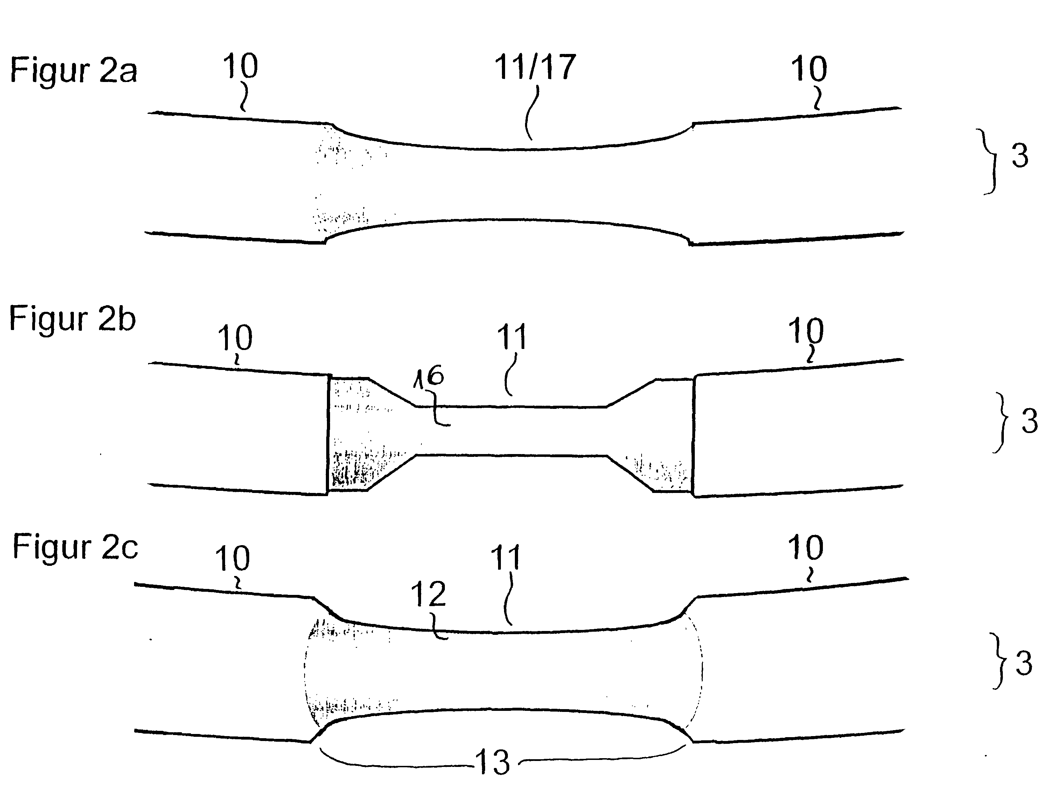

Die Figuren 2a bis c stellen in gegenüber Figur 1 stärkerer Vergrößerung einen Ausschnitt dreier unterschiedlicher Ausführungsformen der erfindungsgemäßen Mikrowendel 3 dar.

Die Figur 2a zeigt eine Mikrowendel 3 mit nicht-korrodierbaren Abschnitten 10 aus einer Platinmetall-Legierung, an die schweißtechnisch eine aus Edelstahl bestehende die elektrolytisch korrodierbare Stelle 11 bildendes Folrmteil 17 von 0,03 mm Durchmesser angefügt ist.

Die Figur 2b zeigt einen Ausschnitt aus einer erfindungsgemäßen Mikrowendel 3 mit einem Mikrosystembaustein 16 als elektrolytisch korrodierbare Stelle 11, welcher durch einen Klebevorgang zwischen die nicht elektrolytisch korrodierbaren Abschnitte 10 eingefügt ist.

Die Figur 2c stellt einen Ausschnitt einer einen Edelstahlkern 12 von 0,03 mm Durchmesser enthaltenden Mikrowendel 3 dar. Dieser Edelstahlkern 12 ist von einer Beschichtung aus elektrolytisch-korrosionsbeständigem Material 13 umgeben, die in regelmäßigen Abständen mit Unterbrechungen versehen ist, an denen der Edelstahlkern 12 von außen zugänglich ist und dementsprechend eine elektrolytisch korrodierbare Stelle 11 ausbildet.

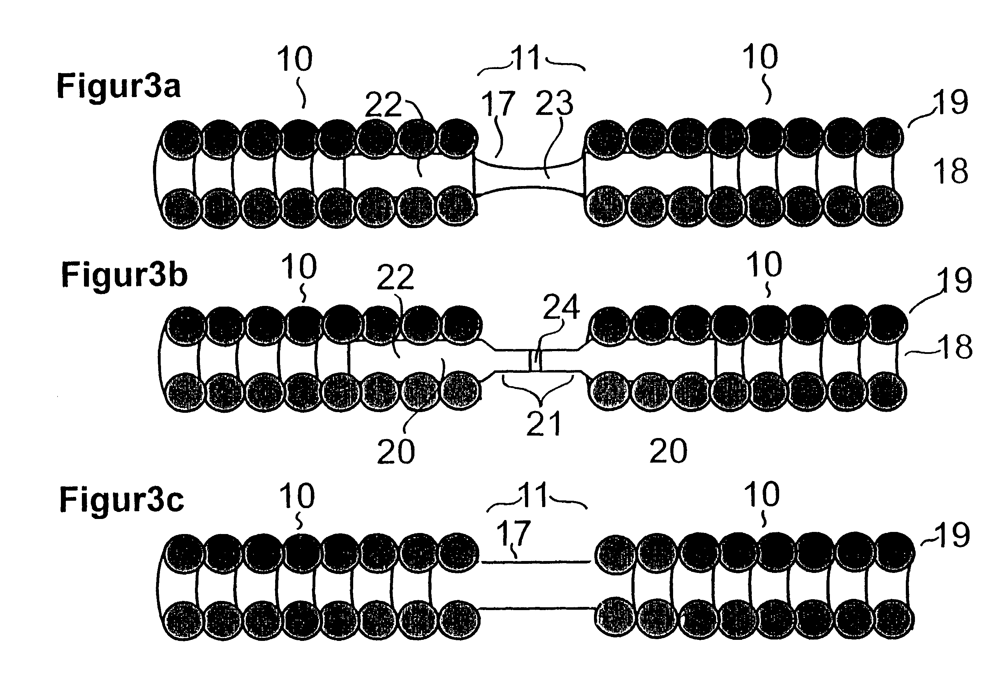

Die Figuren 3a-c stellen in gegenüber Figur 1 stärkerer Vergrößerung einen Ausschnitt dreier unterschiedlicher Ausführungsformen der erfindungsgemäßen Mikrowendel 3 mit in der Längsachse der Mikrowendel 3 ausgerichteten elektrolytisch korrodierbaren Stellen 11 dar.

Die Figur 3a zeigt eine Mikrowendel 3, mit einem im wesentlichen geraden Formteil 17 aus nicht rostendem Stahl, welches formschlüssig in den Innenraum 18 der Mikrowindungen 19 aus Platindraht eingefügt ist. Die modulare Aneinanderfügung von elektrolytisch nicht korrodierbaren Abschnitten 10 aus Platindraht-Mikrowindungen 19 und im wesentlichen geraden Formteilen 17 führt in regelmäßigen Abständen dort, wo die im wesentlichen geraden Formteile 17 nicht von den Platinmetall-Mikrowindungen 19 umgeben und somit von außen zugänglich sind, zur Ausbildung von elektrolytisch korrodierbaren Stellen 11. Die nicht korrodierbaren Abschnitte 10 der Mikrowendel 3 werden dagegen durch den in Mikrowindungen 19 gewundenen Platindraht gebildet, welcher sich jeweils mechanisch formschlüssig zu beiden Seiten um die im wesentlichen gerade ausgebildeten Edelstahlformteile 17 schließt. Das Formteil 17 ist von einer Sn-Schicht 22 umgeben, welche in der Mitte infolge der Vorkorrodierung abgelöst ist. Infolge dessen ist der der elektrolytisch korrodierbare Stelle 11 ausbildende vorkorrodierte Mittelteil 23 des Formteils 17 der elektrolytischen Korrosion besonders gut zugänglich, da er einen sehr geringen Durchmesser aufweist und zu beiden Seiten infolge der Sn-Beschichtung Lokalelemente ausbildet.

Die Figur 3b stellt ebenfalls eine modular aufgebaute Mikrowendel 3 dar, in der sich die Mikrowindungen 19 des Platindrahtes formschlüssig um die Enden eines aus Edelstahl gefertigten Mikrosystembausteins 16 schließen und so die elektrolytisch nicht korrodierbaren Abschnitte 10 bilden, zwischen denen die freiliegenden Abschnitte des Mikrosystembausteins 16 die elektrolytisch korrodierbare Stelle 11 ausbilden. Die Einfügung der Abschnitte des Mikrosystembausteins 16 mit größerem Durchmesser 20 in den Innenraum 18 der Platindrahtmikrowindungen 19 gewährleistet eine stabile Fixierung der modularen Elemente aneinander. Die Ausbildung der elektrolytisch korrodierbaren Stelle 11 durch den verjüngten Abschnitt 21 des Mikrosystembausteins 16 mit geringerem Durchmessers 21 ermöglicht dagegen ein großes Maß an Flexibilität sowie vorteilhaft gute Korrodierbarkeit der elektrolytisch korrodierbaren Stelle 11.

Diese Korrodierbarkeit wird verstärkt durch den Aufbau des Mikrosystembausteins, welcher zum großen Teil aus einer Sn-Legierung 22 besteht und lediglich in der sich verjüngenden Mitte ein Mikroablöseelement 24 enthält, welches aus nicht rostendem Stahl besteht und eine extrem kleine elektrolytisch korrodierbare Stelle ausbildet. Diese vorteilhafte Ausbildungsform ist extrem leicht korrodierbar und daher besonders gut ablösbar.

In Figur 3c ist alternativ der Ausschnitt aus einer erfindungsgemäßen Mikrowendel 3 mit einem im wesentlichen geraden die elektrolytisch korrodierbare Stelle 11 bildenden Formteil 17 aus Edelstahl gezeigt, das schweißtechnisch an die die nicht korrodierbaren Abschnitte 10 bildenden Mikrowindungen 19 aus Platindraht gefügt ist.

Die Elektronegativitäts-Unterschiede zwischen dem die korrodierbaren Stellen 11 ausbildenden Edelstahl und der die nicht-korrodierbaren Abschnitte 10 ausbildenden Platinmetall-Legierung bewirkt in einem ionischen Medium wie dem Blut bei Anlegen einer elektrischen Spannung die elektrolytische Auflösung der elektrolytisch korrodierbaren Stellen 11.

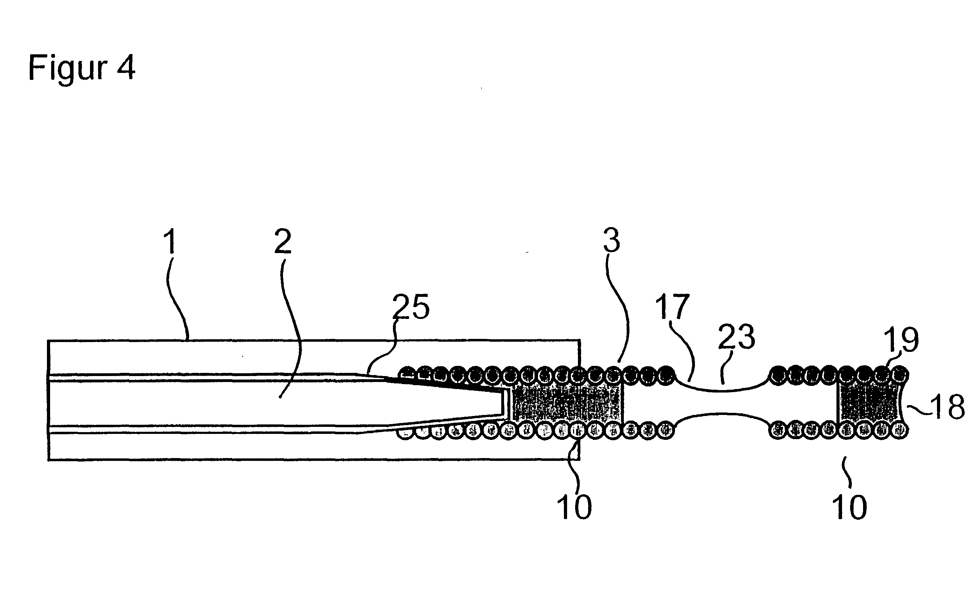

Die Figur 4 stellt einen Längsschnitt durch den Übergang zwischen dem Führungsdraht 2 und der Occlusionswendel 3 in Vergrößerung dar. In diesem Beispiel ist der Führungsdraht 2 aus stabilem nicht rostendem Stahl ausgebildet und von einer Beschichtung 25 umgeben, welche die Korrosion des Stahls verhindert. Diese Beschichtung kann entweder nicht leitend ausgebildet sein, in diesem Falle wird der elektrische Strom über das Ende des Mikrokatheters 1 an die Occlusionswendel 3 geleitet. Oder die Beschichtung ist nicht korrodierbar aber elektrisch leitend (beispielsweise eine Graphitbeschichtung) in welchem Fall der elektrische Strom auch über den Führungsdraht 2 in die Occlusionswendel 3 geleitet werden kann. In diesem Beispiel ist die Occlusionswendel 3 durch eine Anordnung von vorkorrodierten Formteilen 17 aus nicht rostendem Stahl gebildet, welche mechanisch und mit Hilfe von Verkleben in den Innenraum 18 von die elektrolytisch nicht korrodierbaren Stellen bildenden Platindraht Mikrowindungen 19 gefügt sind. In der Mitte vorkorrodierten Formteile 17 bilden aufgrund ihres geringen Durchmessers elektrolytisch besonders gut korrodierbare Stellen aus.

## Patentansprüche

1. Vorrichtung zur Implantation von durch Elektrolyse ablösbaren Occlusionswendeln in Körperhohlräumen oder Blutgefäßen mit einer Spannungsquelle (7), einer Kathode (8), einem Katheter (1) und einer in dem Katheter (1) in Längsrichtung verschiebbaren als Anode dienenden Occlusionswendel (3), wobei die Occlusionswendel (3) an mehreren in Abständen voneinander angeordneten Stellen elektrolytisch korrodierbar ausgebildet ist, so daß im Kontakt mit einer Körperflüssigkeit eine oder mehrere variabel dimensionierte Längen der Occlusionswendel (3) durch Elektrolyse abgetrennt werden können, **dadurch gekennzeichnet, daß** die Occlusionswendel (3) von proximal bis distal durchgehend elektrisch leitend ausgebildet ist.

2. Vorrichtung nach Anspruch 1, **dadurch gekennzeichnet, daß** sich proximal an die Occlusionswendel (3) eine als Führungsdraht (2) ausgebildete Einführhilfe anschließt.

3. Vorrichtung nach Anspruch 2, **dadurch gekennzeichnet, daß** Führungsdraht (2) und Occlusionswendel (3) durch Löt- und/oder Klebe- und/oder Schweißvorgänge und/oder mechanische Verbindungen miteinander verbunden sind.

4. Vorrichtung nach Anspruch 2, **dadurch gekennzeichnet, daß** Führungsdraht (2) und Occlusionswendel (3) als Teile desselben Drahtes ausgebildet sind.

5. Vorrichtung nach einem der vorhergehenden Ansprüche, **dadurch gekennzeichnet, daß** die Occlusionswendel (3) oder ein Teil derselben als Mikrowendel ausgebildet ist.

6. Vorrichtung nach einem der vorhergehenden Ansprüche, **dadurch gekennzeichnet, daß** die Occlusionswendel (3) oder ein Teil derselben unter elastischer Vorspannung steht und nach Entlassen aus dem Katheter (1) Windungen (4) ausbildet.

7. Vorrichtung nach einem der vorhergehenden Ansprüche, **dadurch gekennzeichnet, daß** die elektrolytisch nicht-korrodierbaren Abschnitte (10) der Occlusionswendel (3) eines oder mehrere der folgenden Materialien enthalten: Edelmetalle oder Edelmetall-Legierungen, korrosionsbeständige Keramikwerkstoffe, korrosionsbeständige Kunststoffe, vorzugsweise Platinmetall-Legierungen.

8. Vorrichtung nach einem der vorhergehenden Ansprüche, **dadurch gekennzeichnet, daß** die elektrolytisch korrodierbaren Stellen (10) der Occlusionswendel (3) eines oder mehrere der folgenden Materialien enthalten: nicht korrosionsbeständige Keramikwerkstoffe, nicht korrosionsbeständige Kunststoffe, NichtEdelmetalle oder Legierungen derselben, vorzugsweise nicht rostendem Stahl.

9. Vorrichtung nach Anspruch 8, **dadurch gekennzeichnet, daß** nicht rostender Stahl der Typen AISI 301, 304 oder 316 oder Untergruppen zur Ausbildung der elektrolytisch korrodierbaren Stellen (11) verwendet wird.

10. Vorrichtung nach einem der vorhergehenden Ansprüche, **dadurch gekennzeichnet, daß** für die Ausbildung der elektrolytisch nicht-korrodierbaren Abschnitte (10) an den Übergängen zu den elektrolytisch korrodierbaren Stellen (11) Materialkombinationen gewählt werden, die dazu geeignet sind, Lokalelemente auszubilden.

11. Vorrichtung nach Anspruch 10, **dadurch gekennzeichnet, daß** folgende Materialkombinationen eingesetzt werden:
a) nicht rostende Stähle, vorzugsweise vom Typ AISI 301, 304, 316 oder Untergruppen für die Ausbildung der elektrolytisch korrodierbaren Stellen (11) mit Edelmetallen oder Edelmetall-Legierungen, vorzugsweise Pt oder Pt-Metallen, Pt-Legierungen, Au-Legierungen oder Sn-Legierungen zur Ausbildung der elektrolytisch nicht korrodierbaren Abschnitte (10);
b) Ti oder TiNi-Legierungen zur Ausbildung der elektrolytisch korrodierbaren Stellen (11) mit Edelmetallen oder Edelmetall-Legierungen, vorzugsweise Pt, Pt-Metallen, Pt-Legierungen, Au-Legierungen oder Sn-Legierungen zur Ausbildung der elektrolytisch nicht korrodierbaren Abschnitte (10);
c) Co-Basislegierungen zur Ausbildung der elektrolytisch korrodierbaren Stellen (11) mit Edelmetallen oder Edelmetall-Legierungen, vorzugsweise Pt, Pt-Metallen, Pt-Legierungen, Au-Legierungen oder Sn-Legierungen zur Ausbildung der elektrolytisch nicht korrodierbaren Abschnitte (10).

12. Vorrichtung nach einem der Ansprüche 5 bis 11, **dadurch gekennzeichnet, daß** die Form der elektrolytisch korrodierbaren Stellen (11) an die Mikrowindungen der Mikrowendel (3) angepaßt ist.

13. Vorrichtung nach einem der Ansprüche 1 bis 11, **dadurch gekennzeichnet, daß** die elektrolytisch korrodierbaren Stellen (11) an die Mikrowindungen der Mikrowendel (3) angepaßt ist.

14. Vorrichtung nach einem der Ansprüche 1 bis 11 oder 13, **dadurch gekennzeichnet, daß** die elektrolytisch korrodierbaren Stellen an der Längsachse der Occlusionswendel ausgerichtet sind.

15. Vorrichtung nach einem der vorhergehenden Ansprüche, **dadurch gekennzeichnet, daß** die elektrolytisch korrodierbaren Stellen (11) durch Formteile (17) gebildet werden, die zwischen die elektrolytisch nicht korrodierbaren Abschnitte (10) der Occlusionswendel (3) gefügt werden.

16. Vorrichtung nach Anspruch 15, **dadurch gekennzeichnet, daß** die die elektrolytisch korrodierbaren Stellen (11) bildenden Formteile (17) mit den nicht-korrodierbaren Abschnitten (10) durch Löt- und/oder Klebe- und/oder Schweißvorgänge und/oder mechanische Verfahren verbunden sind.

17. Vorrichtung nach einem der vorhergehenden Ansprüche, **dadurch gekennzeichnet, daß** die elektrolytisch korrodierbaren Stellen (11) vorkorrodiert sind.

18. Vorrichtung nach der vorhergehenden Ansprüche 1-16, **dadurch gekennzeichnet, daß** die elektrolytisch korrodierbaren Stellen (11) bildenden Formteile (17) vorkorrodiert sind.

19. Vorrichtung nach einem der Ansprüche 15 bis 18, **dadurch gekennzeichnet, daß** die Formteile teilweise mit einem Material beschichtet sind, welches in der Spannungsreihe über den die Formteile bildenden Material stehen.

20. Vorrichtung nach Anspruch 19, **dadurch gekennzeichnet, daß** die Formteile aus nicht rostendem Stahl bestehen und die Beschichtung aus Zn, Zn-Legierungen, Sn oder Sn-Legierungen.

21. Vorrichtung nach einem der Ansprüche 15 oder 20, **dadurch gekennzeichnet, daß** die die elektrolytisch korrodierbaren Stellen (11) bildenden Formteile (17) Mikrosystembausteine (16) sind.

22. Vorrichtung nach Anspruch 21, **dadurch gekennzeichnet, daß** die Mikrosystembausteine Mikroablösestellen (24) als elektrolytisch korrodierbare Stellen (11) enthalten.

23. Vorrichtung nach einem der Ansprüche 1 bis 14 , **dadurch gekennzeichnet, daß** die Occlusionswendel (3) oder ein Teil derselben einen durchgängigen Drahtkern aus elektrolytisch korrodierbarem Material enthält, welcher von einer in Längsachse in Abständen wiederholt unterbrochenen Umhüllung aus elektrolytisch korrosionsbeständigem Material (13) umgeben ist.

24. Vorrichtung nach einem der vorhergehenden Ansprüche, **dadurch gekennzeichnet, daß** der Durchmesser der elektrolytisch korrodierbaren Stellen (11) zwischen 0,01 bis 0,05 mm, vorzugsweise 0,02 bis 0,04 mm und besonders bevorzugt 0,03 mm beträgt.

25. Vorrichtung nach einem der vorhergehenden Ansprüche, **dadurch gekennzeichnet, daß** die Drahtspitze des Führungsdrahtes (2) mit einer die Korrosion des Führungsdrahtes verhindernden Beschichtung (25) versehen ist.

26. Vorrichtung nach einem der vorhergehenden Ansprüche **dadurch gekennzeichnet, daß** sie zur Verwendung in Tier- oder humanmedizinischen Verfahren, vorzugsweise bei der Thrombosierung, ausgebildet ist.

## Claims

1. A device for the implantation of occluding coils able to be severed by electrolysis in body cavities or blood vessels comprising a source of electrical power (7), a cathode (8), a catheter (1) and an occluding coil (3) adapted to serve as an anode and able to slide in the catheter (1) in the longitudinal direction, wherein the occluding coil (3) is designed to be electrolytically corrodible at a plurality of spaced apart points so that when in contact with a body fluid one or more variably dimensioned lengths of the occluding coil (3) may be severed by electrolysis, **characterized in that** the occluding coil (3)is designed to be continuously electrically conductive in the proximal-distal direction.

2. The device as set forth in claim 1, **characterized in that** an insertion means, in the form of a guide wire (2), proximally adjoins the occluding coil (3).

3. The device as set forth in claim 2, **characterized in that** the guide wire (2) and the occluding coil (3) are joined together by soldering, brazing and/or bonding and/or welding operations and/or by mechanical connections.

4. The device as set forth in claim 2, **characterized in that** the guide wire (2) and the occluding coil (3) are manufactured in the form of parts of the same wire.

5. The device as set forth in any one of the proceeding claims, **characterized in that** the occluding coil (3) or a part thereof is in the form of a micro-coil.

6. The device as set forth in any one of the proceeding claims, **characterized in that** the occluding coil (3) or a part thereof is subject to a biasing stress and after release from the catheter (1) forms windings.

7. The device as set forth in any one of the proceeding claims, **characterized in that** the electrolytically non-corrodible sections (10) of the occluding coil (3) comprise one or more of the following materials: noble metals or noble meal alloys, corrosion-resistant ceramic materials, corrosion-resistant plastic, and preferably platinum metal alloys.

8. The device as set forth in any one of the proceeding claims, **characterized in that** the electrolytically corrodible points (10) of the occluding coil (3) comprise one or more of the following materials: non-corrosion-resistant ceramic materials, non-corrosion-resistant plastics, base metals or alloys thereof and preferably stainless steel.

9. The device as set forth in claim 8, **characterized in that** stainless steel of the types AISI 301, 304 or 316 or, respectively, subgroups of these types, are employed for the formation of the electrolytically corrodible points (11).

10. The device as set forth in any one of the proceeding claims, **characterized in that** for forming the electrolytically non-corrodible sections (10) adjacent to transitions to the electrolytically corrodible points (11) combinations of materials are selected which are suitable for forming local elements.

11. The device as set forth in claim 10 , **characterized in that** the following combinations of materials are utilized:
a) stainless steels, preferably of the type AISI 301, 304, 316 or subgroups for the formation of the electrolytically corrodible points (11) with noble metals, preferably Pt or Pt metals, Pt alloys, Au alloys or Sn alloys for the formation of the electrolytically non-corrodible sections (10);
b) Ti or TiNi alloys for the formation of the electrolytically corrodible points (11) with noble metals or noble alloys, preferably Pt, Pt metals, Pt alloys, Au alloys or Sn alloys for the formation of the electrolytically non-corrodible sections (10);
c) Co based alloys for the formation of the electrolytically corrodible point (11) with noble metal or noble metal alloys, preferably Pt, Pt metals, Pt alloys, Au alloys or Sn alloys for the formation of the electrolytically non-corrodible sections (10).

12. The device as set forth in any one of the claims 5 through 11, **characterized in that** the shape of the electrolytically corrodible points (11) is adapted to the micro-windings of the micro-coil (3).

13. The device as set forth in any one of the claims 1 through 11, **characterized in that** the electrolytically corrodible points (11) are adapted to the micro-windings of the micro-coil (3).

14. The device as set forth in any one of the claims 1 through 11 or claim 13, **characterized in that** the electrolytically corrodible points are aligned with the longitudinal axis of the occluding coil.

15. The device as set forth in any one of the proceeding claims, **characterized in that** the electrolytically corrodible points (11) are constituted by fittings (17) which are fitted between the electrolytically non-corrodible sections (10) of the occluding coil (3).

16. The device as set forth in claim 15, **characterized in that** the fittings (17) constituting the electrolytically corrodible points (11) are joined to the electrolytically non-corrodible sections (10) by soldering and/or brazing and/or bonding and/or welding operations and/or mechanical methods.

17. The device as set forth in any one of the proceeding claims, **characterized in that** the electrolytically corrodible points (11) are pre-corroded.

18. The device as set forth in any one of the proceeding claims 1 through 16, **characterized in that** the fittings (17) constituting the electrolytically corrodible points (11) are pre-corroded.

19. The device as set forth in any one of the proceeding claims 15 through 18, **characterized in that** the fittings are partly coated with a material, which is higher up in the electrochemical series than the material forming the fittings.

20. The device as set forth in claim 19, **characterized in that** the fittings consist of stainless steel and the coating consists of Zn, Zn alloys, Sn or Sn alloys.

21. The device as set forth in any one of the proceeding claims 15 through 20, **characterized in that** the fittings (17) constituting the electrolytically corrodible points (11) are micro-system components (16).

22. The device as set forth in claim 21, **characterized in that** the micro-system components contain micro-severance points (24) as electrolytically corrodible points (11).

23. The device as set forth in any one of the claims 1 through 14, **characterized in that** the occluding coil (3) or a part thereof contains a continuous wire core of electrolytically corrodible material, which is surrounded by a casing repeatedly interrupted at intervals along the longitudinal axis and made of electrolytically corrosion resistant material (13).

24. The device as set forth in any one of the proceeding claims, **characterized in that** the diameter of the electrolytically corrodible points (11) is between 0.01 and 0.05 mm, preferably 0.02 and 0.04 and more particularly 0.03 mm.

25. The device as set forth in any one of the proceeding claims, **characterized in that** the end of the guide wire (2) is provided with a coating (25) preventing corrosion of the guide wire.

26. The device as set forth in any one of the proceeding claims, **characterized in that** it is adapted for methods used in veterinary or human medicine and preferably for thrombozation.

## Revendications

1. Dispositif pour l'implantation d'éléments hélicoïdaux d'occlusion détachables par électrolyse dans des cavités corporelles ou des vaisseaux sanguins avec une source de tension (7), une cathode (8), un cathéter (1) et un élément hélicoïdal d'occlusion (3) servant d'anode et mobile dans la direction longitudinale dans le cathéter (1), l'élément hélicoïdal d'occlusion (3) étant réalisable corrodable électrolytiquement en plus dans des endroits espacés les uns des autres, de sorte qu'en contact avec un liquide corporel, une ou plusieurs longueurs variables de l'élément hélicoïdal d'occlusion (3) peut/peuvent être séparée(s) par électrolyse, **caractérisé en ce que** l'élément hélicoïdal d'occlusion (3) est réalisé conducteur électrique, de manière continue, de son extrémité proximale à son extrémité distale.

2. Dispositif selon la revendication 1, **caractérisé en ce que**, du côté proximal, l'élément hélicoïdal d'occlusion (3) est suivi d'une aide à l'introduction conformée en fil de guidage (2).

3. Dispositif selon la revendication 2, **caractérisé en ce que** le fil de guidage (2) et l'élément hélicoïdal d'occlusion (3) sont reliés entre eux par des opérations de brasage et/ou collage et/ou soudage et/ou par des liaisons mécaniques.

4. Dispositif selon la revendication 2, **caractérisé en ce que** le fil de guidage (2) et l'élément hélicoïdal d'occlusion (3) sont formés en partie du même fil.

5. Dispositif selon l'une des revendications précédentes, **caractérisé en ce que** l'élément hélicoïdal d'occlusion (3) ou une partie de celui-ci est conformé en micro-spire.

6. Dispositif selon l'une des revendications précédentes, **caractérisé en ce que** l'élément hélicoïdal d'occlusion (3) ou une partie de celui-ci est sous précontrainte élastique et forme des enroulements (4) à sa sortie du cathéter (1).

7. Dispositif selon l'une des revendications précédentes, **caractérisé en ce que** les sections (10) non-corrodables électrolytiquement de l'élément hélicoïdal d'occlusion (3) contiennent un ou plusieurs des matériaux suivants : métaux nobles ou alliages de métaux nobles, matériaux céramiques résistant à la corrosion, matières plastiques résistant à la corrosion, de préférence des alliages platine-métal.

8. Dispositif selon l'une des revendications précédentes, **caractérisé en ce que** les parties (10) corrodables électrolytiquement de l'élément hélicoïdal d'occlusion (3) contiennent un ou plusieurs des matériaux suivants : matériaux céramiques ne résistant pas à la corrosion, matières plastiques ne résistant pas à la corrosion, métaux non nobles ou leurs alliages, de préférence de l'acier inoxydable.

9. Dispositif selon la revendication 8, **caractérisé en ce que** l'on utilise un acier inoxydable des types AISI 301, 304 ou 316 ou leurs sous-groupes pour réaliser les parties (11) corrodables électrolytiquement.

10. Dispositif selon l'une des revendications précédentes, **caractérisé en ce que**, pour réaliser les parties (10) non-corrodables électrolytiquement, on choisit au niveau des transitions avec les parties corrodables électrolytiquement (11) des combinaisons de matériaux qui conviennent pour former des éléments locaux.

11. Dispositif selon la revendication 10, **caractérisé en ce que** l'on utilise les combinaisons de matériaux suivantes :
a) aciers inoxydables, de préférence de type AISI 301, 304, 316 ou leurs sous-groupes pour la formation des parties (11) corrodables électrolytiquement avec des métaux nobles ou des alliages de métaux nobles, de préférence Pt ou Pt-métaux, alliages de Pt, alliages d'Au ou alliages de Sn pour la formation des sections (10) non-corrodables électrolytiquement ;
b) Ti ou alliages de TiNi pour la formation des parties corrodables électrolytiquement (11) avec des métaux nobles ou des alliages de métaux nobles, de préférence Pt ou Pt-métaux, alliages de Pt, alliages d'Au ou alliages de Sn pour la formation des sections (10) non-corrodables électrolytiquement ;
c) alliages à base de Co pour la formation des parties corrodables électrolytiquement (11) avec des métaux nobles ou des alliages de métaux nobles, de préférence Pt ou Pt-métaux, alliages de Pt, alliages d'Au ou alliages de Sn pour la formation des sections (10) non-corrodables électrolytiquement.

12. Dispositif selon l'une des revendications 5 à 11, **caractérisé en ce que** la forme des parties (11) corrodables électrolytiquement est adaptée aux micro-enroulements de la micro-spire (3).

13. Dispositif selon l'une des revendications 1 à 11, **caractérisé en ce que** les parties (11) corrodables électrolytiquement sont adaptées aux micro-enroulements de la micro-spire (3).

14. Dispositif selon l'une des revendications 1 à 11 ou 13, **caractérisé en ce que** les parties corrodables électrolytiquement sont orientées selon l'axe longitudinal de l'élément hélicoïdal d'occlusion.

15. Dispositif selon l'une des revendications précédentes, **caractérisé en ce que** les parties (11) corrodables électrolytiquement sont formées par des pièces moulées (17) qui sont insérées entre les sections (10) corrodables électrolytiquement de l'élément hélicoïdal d'occlusion (3).

16. Dispositif selon la revendication 15, **caractérisé en ce que** les pièces moulées (17) formant parties (11) corrodables électrolytiquement sont reliées aux sections (10) non-corrodables électrolytiquement par des opérations de brasage et/ou collage et/ou soudage et/ou par des liaisons mécaniques.

17. Dispositif selon l'une des revendications précédentes, **caractérisé en ce que** les parties (11) corrodables électrolytiquement sont pré-corrodées.

18. Dispositif selon l'une des revendications 1-16, **caractérisé en ce que** les pièces moulées (17) formant les parties (11) corrodables électrolytiquement sont pré-corrodées.

19. Dispositif selon l'une des revendications 15 à 18, **caractérisé en ce que** les pièces moulées sont partiellement revêtues d'un matériau qui, dans la série des tensions, se trouve au-dessus du matériau formant les pièces moulées.

20. Dispositif selon la revendication 19, **caractérisé en ce que** les pièces moulées ne sont pas en acier inoxydable et le revêtement est composé de Zn, alliages de Zn, Sn ou alliages de Sn.

21. Dispositif selon l'une des revendications 15 ou 20, **caractérisé en ce que** pièces moulées (17) formant les parties (11) corrodables électrolytiquement sont des microcomposants de système (16).

22. Dispositif selon la revendication 21, **caractérisé en ce que** les microcomposants de système contiennent des micro-zones de détachement (24) servant de parties corrodables électrolytiquement (11).

23. Dispositif selon l'une des revendications 1 à 14, **caractérisé en ce que** l'élément hélicoïdal d'occlusion (3) ou une partie de celui-ci contient une âme de fil continue en matériau corrodable électrolytiquement, qui est entouré par une gaine, interrompue plusieurs fois à distance le long de l'axe longitudinal, en matériau (13) non corrodable électrolytiquement.

24. Dispositif selon l'une des revendications précédentes, **caractérisé en ce que** le diamètre des parties corrodables électrolytiquement (11) est compris entre 0,01 et 0,05 mm, de préférence entre 0,02 et 0,04 mm, et vaut, de manière particulièrement préférée, 0,03 mm.

25. Dispositif selon l'une des revendications précédentes, **caractérisé en ce que** l'extrémité du fil de guidage (2) est munie d'un revêtement (25) empêchant la corrosion du fil de guidage.

26. Dispositif selon l'une des revendications précédentes, **caractérisé en ce qu'**il est conformé en vue d'une utilisation dans des procédures de médecine humaine ou animale, de préférence dans le traitement des thromboses.
